# DEMANDE DE BREVET EUROPEEN

(11) **EP 1 647 281 A2**
(43) Date de publication de la demande: **19.04.2006**
(21) Numéro de dépôt: 05025200.6
(22) Date de dépôt: 15.07.1997
(51) Int. Cl.: A61K 39/295, C12N 15/38, C12N 15/44, C12N 15/31

(54) **Formule de vaccin polynucléotidique contre les pathologies du cheval**

(30) Priorité: 19.07.1996 FR 9609400
(62) Demande divisionnaire de: 97934569.1
(71) Demandeur: MERIAL, 69007 Lyon (FR)
(72) Inventeur: Audonnet, Jean-Christophe, 69006 Lyon (FR); Bouchardon, Annabelle, 69007 Lyon (FR); Riviere, Michel, 69130 Ecully (FR)
(74) Mandataire: Nargolwalla, Cyra

(57) **Abrégé**

La présente invention est relative à une formule de vaccin pour équidés, notamment des chevaux, comprenant un ou plusieurs plasmides intégrant, de manière à l'exprimer *in vivo* dans les cellules au moins un gène codant pour un antigène du virus de l'encéphalite.

## Description

La présente invention est relative à une formule de vaccin permettant la vaccination des équidés, notamment des chevaux. Elle est également relative à une méthode de vaccination correspondante.

La pathologie des chevaux présente une assez grande diversité. Outre les pathologies respiratoires bien connues, telles que la rhinopneumonie et la grippe, les chevaux sont sensibles, notamment sur le continent américain, aux diverses encéphalites. Enfin, les chevaux présentent diverses autres pathologies parmi lesquelles on peut citer, notamment, le tétanos, la maladie de Lyme, l'artérite équine, sans oublier les risques d'exposition au virus de la rage.

Les conditions d'exposition des chevaux aux divers microorganismes pathogènes ont été multipliées par les déplacements de nombreux chevaux sur les distances importantes par voie de terre ou par voie aérienne, de sorte que le risque infectieux a tendance à augmenter.

Or, compte-tenu du coût élevé de ces animaux, notamment dans le cas des reproducteurs, des chevaux de selle et des chevaux de course, il est économiquement important de contrôler le plus possible les risques infectieux se traduisant par de longues indisponibilités de l'animal, voir sa perte. Il existe déjà un certain nombre de vaccins pour chevaux, dont l'efficacité est variable.

Ainsi, ont été développés, pour la rhinopneumonie équine, provoquée par les différentes souches d'herpès virus équin (EHV), des vaccins inactivés ou de sous-unités, qui présentent cependant tous un certain nombre de limites se traduisant par des protections incomplètes et de courte durée et éventuellement des problèmes d'innocuité liés aux adjuvants utilisés.

La grippe équine constitue également une pathologie importante, que l'on cherche, également à prévenir par la vaccination. Les vaccins utilisés sont des vaccins inactivés ou de sous-unités, qui présentent une certaine efficacité mais qui ne restent pas néanmoins sans problème. En effet, il est fréquent que la protection ne soit pas complète et elle est généralement d'une durée relativement brève, nécessitant, comme pour la rhinopneumonie, des revaccinations. On peut aussi rencontrer des problèmes d'innocuité.

Des vaccins à base d'anatoxine antitétanique ont également été mis au point et présentent une efficacité indéniable.

Il existe également des vaccins contre les encéphalites, quelques encéphalites de l'Est, l'encéphalite de l'Ouest et l'encéphalite vénézuélienne, dont l'efficacité est encore mal connue.

Pour des raisons tenant d'une part à l'économie, et d'autre part à la gestion rationnelle des vaccinations équines, on a déjà proposé des formulations de vaccin multivalent pour la prévention de plusieurs de ces maladies infectieuses.

Les associations développées jusqu'à présent étaient réalisées à partir de vaccins inactivés ou de vaccins vivants et éventuellement de mélanges de tels vaccins. Leur mise en oeuvre pose des problèmes de compatibilité entre valences et de stabilité. Il faut en effet assurer à la fois la compatibilité entre les différentes valences, que ce soit au plan des différents antigènes utilisés et au plan des formulations elles-mêmes, notamment dans le cas où l'on combine à la fois des vaccins inactivés et des vaccins vivants. Il se pose également le problème de la conservation de tels vaccins combinés et aussi de leur innocuité notamment en présence d'adjuvant. Ces vaccins sont en général assez coûteux.

En outre, ces formulations n'ont pas permis d'associer trois des valences majeures, à savoir les valences grippe équine, rhinopneumonie, notamment EHV-1 et EHV-4, et tétanos. On connaît, par exemple, les associations des valences grippes et encéphalites, ou rhinopneumie et encéphalites.

Les demandes de brevet WO-A-90 11 092, WO-A-93 19 183, WO-A-94 21 797 et WO-A-95 20 660 ont proposé de faire usage de la technique récemment développée des vaccins polynucléotidiques. On sait que ces vaccins utilisent un plasmide apte à exprimer dans les cellules de l'hôte l'antigène inséré dans le plasmide.

Toutes les voies d'administration ont été proposées (intrapéritonéale, -intraveineuse, intramusculaire, transcutanée, intradermique, mucosale, etc.). Différents moyens de vaccination peuvent également être utilisés, tels que ADN déposé à la surface de particules d'or et projeté de façon à pénétrer dans la peau de l'animal (Tang et al., Nature 356, 152-154, 1992) et les injecteurs par jet liquide permettant d'assurer la transfection à la fois dans la peau, le muscle, les tissus graisseux, et les tissus mammaires (Furth et al., Analytical Biochemistry, 205, 365-368, 1992).

Les vaccins polynucléotidiques peuvent utiliser aussi bien des ADN nus que des ADN formulés, par exemple au sein des liposomes ou de lipides cationiques.

Des vecteurs polynucléotidiques, intégrant les gènes HA ou NP, ont été essayés pour le virus de la grippe (influenza virus) chez la souris, le furet et le poulet. Aucune donnée n'est disponible chez le cheval.

Concernant le tétanos, il a été rapporté, récemment, que l'immunisation de souris par un plasmide exprimant, avec le fragment C, la région C-terminale non toxique de la toxine tétanique, induisait l'apparition d'anticorps séro-protecteurs chez la souris.

Il n'est cependant pas possible de transposer directement les enseignements des résultats obtenus chez ces animaux de faibles longévités à celui d'autres mammifères, et notamment les mammifères de grandes tailles.

La protection des équidés et notamment des chevaux contre la pathologie infectieuse demande donc toujours à être améliorée.

L'invention se propose de fournir une formule de vaccin multivalent permettant d'assurer une vaccination des équidés, et notamment des chevaux, contre un certain nombre d'agents pathogènes.

Un autre objectif de l'invention est de fournir une telle formule de vaccin associant différentes valences tout en présentant les critères requis de compatibilité et de stabilité des valences entre elles.

Un autre objectif de l'invention est de fournir une telle formule de vaccin permettant d'associer différentes valences dans un même véhicule.

Un autre objectif de l'invention est de fournir une telle formule de vaccin qui soit de mise en oeuvre aisée et peu coûteuse.

Un autre objectif encore de l'invention est de fournir une telle formule et une méthode de vaccination des chevaux qui permette d'obtenir une protection, y compris une protection multivalente, avec un niveau élevé d'efficacité et de longue durée, et une bonne innocuité.

La présente invention a donc pour objet une formule de vaccin contre des pathologies des équidés et notamment des chevaux, comprenant au moins 3 valences de vaccin polynucléotidique comprenant chacune un plasmide intégrant, de manière à l'exprimer in vivo dans les cellules, un gène d'une valence de pathogène équin, ces valences étant choisies parmi celles du groupe consistant en virus de la rhinopneumonie équine EHV, virus de la grippe équine EIV et le tétanos (C1. tetani), ces plasmides comprenant, pour chaque valence, un ou plusieurs des gènes choisis parmi le groupe consistant en gB et gD pour le virus de la rhinopneumonie équine, HA, NP, N pour le virus de la grippe équine et un gène codant pour tout ou partie de la sous-unité C de la toxine tétanique.

Par valence, dans la présente invention, on entend au moins un antigène assurant une protection contre le virus du pathogène considéré, la valence pouvant contenir, à titre de sous-valence, un ou plusieurs gènes naturels ou modifiés d'une ou plusieurs souches du pathogène considéré.

Par gène d'agent pathogène, on entend non seulement le gène complet, mais aussi les séquences nucléotidiques différentes, y compris fragments, conservant la capacité à induire une réponse protectrice. La notion de gène recouvre les séquences nucléotidiques équivalentes à celles décrites précisément dans les exemples, c'est-à-dire les séquences différentes mais codant pour la même protéine. Elle recouvre aussi les séquences nucléotidiques d'autres souches du pathogène considéré, assurant une protection croisée ou une protection spécifique de souche ou de groupe de souche. Elle recouvre encore les séquences nucléotidiques qui ont été modifiées pour faciliter l'expression in vivo par l'animal hôte mais codant pour la même protéine.

Ainsi, de manière particulièrement préférée, le vaccin selon l'invention comprend dans la valence rhinopneumonie équine, au moins un antigène de la souche EHV-1 et au moins un antigène de la souche EHV-4, et de préférence le même type d'antigène.

Les quantités thérapeutiquement efficaces des valences polynucléotidiques sont contenues ou destinées à être contenues, dans un véhicule convenable pour une administration à l'animal, et de préférence pour une administration intramusculaire. De préférence, ce véhicule est un véhicule aqueux dépourvu de constituants huileux.

En ce qui concerne la valence rhinopneumonie équine, on préférera associer les gènes gB et gD, de préférence des souches EHV, notamment souches 1 et 4.

En ce qui concerne la valence grippe équine, on préfère utiliser le gène codant pour l'hémagglutinine HA ou l'association des gènes codant pour HA et NP. De préférence, on associera dans le même vaccin, les séquences HA de virus influenza, en particulier des différentes souches rencontrées sur le terrain. En revanche, NP assure une protection croisée et l'on pourra donc se contenter de la séquence d'une seule souche du virus.

Pour la valence tétanos, on préférera la sous-unité C éventuellement modifiée par mutation ou délétion.

L'association de gènes codant pour plusieurs antigènes d'une même valence, ou d'une même souche dans une valence, peut être réalisée par le mélange de plasmide exprimant un seul antigène, ou au contraire en insérant plusieurs gènes dans un même plasmide.

La combinaison des différentes valences du vaccin selon l'invention peut être effectuée, de préférence, par mélange de plasmides polynucléotidiques exprimant un ou des antigènes de chaque valence, mais on peut également prévoir de faire exprimer plusieurs antigènes de plusieurs valences par un même vecteur de type plasmidique.

Dans une forme perfectionnée de l'invention, la formulation peut encore comporter une ou plusieurs autres valences d'autres pathogènes des équins, et notamment des valences des virus de l'encéphalite de l'Est EEV, de l'encéphalite de l'Ouest WEV et de l'encéphalite vénézuélienne VEV, de préférence les trois simultanément.

Parmi ces valences peuvent également figurer avantageusement la valence de la maladie de Lyme, B. burgdorferi, de l'artérite équine (EAV) et de la rage.

Parmi les gènes des encéphalites précitées, on utilise les gènes des antigènes C et E2, et de préférence le gène E2 seul ou l'association des deux gènes E2 et C.

Dans la valence de la maladie de Lyme, on choisit parmi les gènes OspA, OspB et p100, de préférence OspA.

Pour l'artérite équine, on retiendra les gènes E, M et N, seuls ou associés.

Pour la rage, on retiendra le gène G.

Une formule de vaccin selon l'invention pourra se présenter sous un volume de doses compris entre 0,1 et 10 ml et en particulier entre 1 et 5 ml.

La dose sera généralement comprise entre 10 ng et 1 mg, notamment entre 100 ng et 500 µg, et préférentiellement entre 1 µg et 250 µg par type de plasmide.

On utilisera de préférence des plasmides nus, simplement placés dans le véhicule de vaccination qui sera en général de l'eau physiologique (NaCl 0,9 %), de l'eau ultrapure, du tampon TE, etc. On peut bien entendu utiliser toutes les formes de vaccin polynucléotidique décrites dans l'art antérieur.

Chaque plasmide comprend un promoteur apte à assurer l'expression du gène inséré sous sa dépendance dans les cellules hôtes. Il s'agira, en général, d'un promoteur eucaryote fort, et en particulier d'un promoteur précoce du cytomégalovirus CMV-IE, d'origine humaine ou murine, ou encore . éventuellement d'une autre origine telle que rat, porc, cobaye.

De manière plus générale, le promoteur pourra être soit d'origine virale, soit d'origine cellulaire. Comme protecteur viral autre que CMV-IE, on peut citer le promoteur précoce ou tardif du virus SV 40 ou le promoteur LTR du virus du Sarcome de Rous. Il peut aussi s'agir d'un promoteur du virus dont provient le gène, par exemple le promoteur propre au gène.

Comme promoteur cellulaire, on peut citer le promoteur d'un gène du cytosquelette, tel que par exemple le promoteur de la desmine (Polmont et al., Journal of Submicroscopic Cytology and Pathology, 1990, 22, 117-122 ; et Zhenlin et al., Gene, 1989, 78, 243-254), ou encore le promoteur de l'actine.

Lorsque plusieurs gènes sont présent dans un même plasmide, ceux-ci peuvent être présentés dans la même unité de transcription ou dans deux unités différentes.

L'invention a encore pour objet des formules de vaccin monovalent comprenant un ou plusieurs plasmides codant pour un ou plusieurs gènes de l'un des agents pathogènes précités, et notamment de la rhinopneumonie ou de la maladie de Lyme, de l'artérite équine, de l'encéphalite de l'Est, de l'encéphalite de l'Ouest et de l'encéphalite vénézuélienne, les gènes étant ceux décrits plus haut. Ces formules peuvent comprendre les caractéristiques énoncées plus haut en ce qui concerne le choix des gènes d'un même pathogène et leur combinaison, la composition des plasmides, les volumes de doses, les dosages, etc.

La présente invention a également pour objet une méthode de vaccination des équidés et notamment des chevaux contre des maladies infectieuses, comprenant l'administration d'une dose efficace d'une formule de vaccin, multivalente ou monovalente, telle que décrite plus haut.

Cette méthode de vaccination comprend l'administration d'une ou de plusieurs doses de la formule de vaccin.

Les formules de vaccin selon l'invention pourront être administrées par les différents voies d'administration proposées dans le cadre général de la vaccination polynucléotidique et au moyen des techniques d'administration connues. On préférera cependant nettement la voie intramusculaire.

On peut aussi vacciner par voie intradermique à l'aide d'un injecteur par jet liquide, de préférence par jets multiples, et en particulier un injecteur utilisant une tête d'injection munie de plusieurs trous ou buses, notamment comprenant de 5 à 6 trous ou buses, tel que l'appareil Pigjet fabriqué et distribué par la société Endoscoptic, Laons, France.

Le volume de dose pour un tel appareil sera réduit de préférence entre 0,1 et 0,9 ml, en particulier entre 0,2 et 0,6 ml et avantageusement entre 0,4 et 0,5 ml, le volume pouvant être administré en une ou plusieurs, de préférence 2, applications.

Les vaccins monovalents précités peuvent être notamment utilisés pour la préparation du vaccin polyvalent selon l'invention.

Les formules de vaccin monovalent peuvent aussi être utilisées associées à un vaccin d'un autre type (entier vivant ou inactivé, recombinant, sous-unité) contre une autre pathologie ou comme rappel d'un vaccin comme décrit ci-après.

La présente invention a en effet encore pour objet l'utilisation d'un ou de plusieurs plasmides selon l'invention pour la fabrication d'un vaccin destiné à vacciner des chevaux primo-vaccinés au moyen d'un premier vaccin classique (monovalent ou multi-valent) du type de ceux de l'art antérieur, choisi notamment dans le groupe consistant en vaccin entier vivant, vaccin entier inactivé, vaccin de sous-unité, vaccin recombinant, ce premier vaccin présentant (c'est-à-dire contenant ou pouvant exprimer) le ou les antigène(s) codé(s) par le ou les plasmide(s) ou antigène(s) assurant une protection croisée.

De manière remarquable, le vaccin polynucléotidique a un effet de rappel puissant se traduisant par une amplification de la réponse immunitaire et l'instauration d'une immunité de longue durée.

De manière générale, les vaccins de primo-vaccination pourront être choisis parmi les vaccins commerciaux disponibles auprès des différents producteurs de vaccins vétérinaires.

Dans une forme de mise en oeuvre préférée du procédé selon l'invention, on administre dans un premier temps, à l'animal, une dose efficace du vaccin de type classique, notamment inactivé, vivant, atténué ou recombinant, ou encore un vaccin de sous-unité de façon à assurer une primo-vaccination, et, après un délai de préférence de 2 à 4 semaines, on assure l'administration du vaccin polyvalent ou monovalent selon l'invention.

L'invention a aussi pour objet un kit de vaccination regroupant une formule de vaccin selon l'invention et un vaccin de primo-vaccination tel que décrit ci-dessus. Elle a aussi trait à une formule de vaccin selon l'invention accompagnée d'une notice indiquant l'usage de cette formule comme rappel d'une primo-vaccination telle que décrite ci-avant.

L'invention concerne aussi la méthode de préparation des formules de vaccin, à savoir la préparation des valences et leurs mélanges, telle qu'elle ressort de cette description.

L'invention va être maintenant décrite plus en détail à l'aide de modes de réalisation de l'invention pris en référence aux dessins.

### Liste des figures

- Figure N° 1 :: Plasmide pVR1012
- Figure N° 2 :: Plasmide pAB042
- Figure N° 3 :: Plasmide pAB031
- Figure N° 4 :: Plasmide pAB013
- Figure N° 5 :: Plasmide pAB032
- Figure N° 6 :: Plasmide pAB043
- Figure N° 7 :: Plasmide pAB033
- Figure N° 8 :: Séquence du gène HA Grippe équine souche Fontainebleau
- Figure N° 9 :: Plasmide pAB099
- Figure N° 10 :: Plasmide pAB085
- Figure N° 11 :: Plasmide pAB084
- Figure N° 12 :: Plasmide pAB070
- Figure N° 13 :: Plasmide pAB017
- Figure N° 14 :: Plasmide pAB094
- Figure N° 15 :: Plasmide pAB093
- Figure N° 16 :: Plasmide pAB096
- Figure N° 17 :: Plasmide pAB095
- Figure N° 18 :: Plasmide pAB098
- Figure N° 19 :: Plasmide pAB097
- Figure N° 20 :: Plasmide pAB041

### Liste des séquences SEQ ID N°

- SEQ ID N° 1 :: Oligonucléotide AB013
- SEQ ID N° 2 :: Oligonucléotide AB014
- SEQ ID N° 3 :: Oligonucléotide AB071
- SEQ ID N° 4 :: Oligonucléotide AB074
- SEO ID N° 5 :: Oligonucléotide AB030
- SEQ ID N° 6 :: Oligonucléotide AB031
- SEQ ID N° 7 :: Oligonucléotide AB075
- SEQ ID N° 8 :: Oligonucléotide AB076
- SEQ ID N° 9 :: Oligonucléotide AB015
- SEQ ID N° 10 :: Oligonucléotide AB016
- SEQ ID N° 11 :: Oligonucléotide AB077
- SEQ ID N° 12 :: Oligonucléotide AB078
- SEQ ID N° 13 :: Oligonucléotide AB186
- SEQ ID N° 14 :: Oligonucléotide AB187
- SEQ ID N° 15 :: Séquence du gène HA Grippe équine (souche Fontainebleau)
- SEQ ID N° 16 :: Oligonucléotide AB156
- SEQ ID N° 17 :: Oligonucléotide AB159
- SEQ ID N° 18 :: Oligonucléotide AB157
- SEQ ID N° 19 :: Oligonucléotide AB128
- SEC ID N° 20 :: Oligonucléotide AB129
- SEQ ID N° 21 :: Oligonucléotide AB038
- SEQ ID N° 22 :: Oligonucléotide AB039
- SEQ ID N° 23 :: Oligonucléotide AB176
- SEQ ID N° 24 :: Oligonucléotide AB177
- SEQ ID N° 25 :: Oligonucléotide AB174
- SEQ ID N° 26 :: Oligonucléotide AB175
- SEQ ID N° 27 :: Oligonucléotide AB180
- SEQ ID N° 28 :: Oligonucléotide AB181
- SEQ ID N° 29 :: Oligonucléotide AB178
- SEQ ID N° 30 :: Oligonucléotide AB179
- SEQ ID N° 31 :: Oligonucléotide AB184
- SEQ ID N° 32 :: Oligonucléotide AB185
- SEQ ID N° 33 :: Oligonucléotide AB182
- SEQ ID N° 34 :: Oligonucléotide AB183
- SEQ ID N° 35 :: Oligonucléotide AB011
- SEO ID N° 36 :: Oligonucléotide AB012

### EXEMPLES

### Exemple 1 : Culture des virus

Les virus sont cultivés sur le système cellulaire approprié jusqu'à obtention d'un effet cytopathique. Les systèmes cellulaires à utiliser pour chaque virus sont bien connus de l'homme du métier. Brièvement, des cellules sensibles au virus utilisé, cultivées en milieu minimum essentiel de Eagle (milieu "MEM) ou un autre milieu approprié, sont inoculées avec la souche virale étudiée en utilisant une multiplicité d'infection de 1. Les cellules infectées sont alors incubées à 37°C pendant le temps nécessaire à l'apparition d'un effet cytopathique complet (en moyenne 36 heures).

### Exemple 2 : Culture des bactéries:

Tétanos...........Les bactéries sont cultivées dans les milieux appropriés et selon les conditions bien connues de l'homme de l'art de manière à obtenir une biomasse bactérienne suffisante pour l'extraction du matériel génétique. Cette extraction se fait selon les techniques usuelles décrites par Sambrook J. *et al*. (*Molecular Cloning: A Laboratory Manual*. 2^{nd} Edition. Cold Spring Harbor Laboratory. Cold Spring Harbor. New York. 1989).
Les souches de *Borrelia burgdorferi* sont cultivées dans les milieux appropriés et selon les conditions bien connues de l'homme de l'art. Ces conditions et milieux sont en particulier décrits par A. Barbour (J. Biol. Med. 1984. **57.** 71-75). L'extraction de l'ADN bactérien a été réalisé selon les conditions décrites par W. Simpson et al. (Infect. Immun. 1990. **58.** 847-853). Les techniques usuelles décrites par J. Sambrook *et al*. (*Molecular Cloning: A Laboratory Manual*. 2^{nd} Edition. Cold Spring Harbor Laboratory. Cold Spring Harbor. New York. 1989) peuvent également être utilisées.

### Exemple 3 : Extraction des ADNs génomiques viraux:

Après culture, le surnageant et les cellules lysées sont récoltées et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EDTA 1 mM). Cette suspension virale concentrée est traitée par la protéinase K (100 µg/ml final) en présence de sodium dodecyl sulfate (SDS) (0,5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN est centrifugé à 10000 g pendant 15 minutes à + 4°C. Le culot d'ADN est séché, puis repris dans un volume minimum d'eau ultrapure stérile. Il peut alors être digéré par des enzymes de restriction.

### Exemple 4 : Isolement des ARNs génomiques viraux

Les virus à ARN ont été purifiés selon les techniques bien connues de l'homme du métier. L'ARN viral génomique de chaque virus a été ensuite isolé en utilisant la technique d'extraction "thiocyanate de guanidium/phénolchloroforme" décrite par P. Chomczynski et N. Sacchi (Anal. Biochem. 1987. 162. 156-159).

### Exemple 5 : Techniques de biologie moléculaire

Toutes les constructions de plasmides ont été réalisées en utilisant les techniques standards de biologie moléculaire décrites par J. Sambrook *et al. (Molecular Cloning: A Laboratory Manual*. 2^{nd} Edition. Cold Spring Harbor Laboratory. Cold Spring Harbor. New York. 1989). Tous les fragments de restriction utilisés pour la présente invention ont été isolés en utilisant le kit "Geneclean" (BIO101 Inc. La Jolla, CA).

### Exemple 6 : Technique de RT-PCR

Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (voir exemples spécifiques). La réaction de transcription inverse (RT) et l'amplification en chaîne par polymérase (PCR) ont été effectuées selon les techniques standards (Sambrook J. *et al*. 1989). Chaque réaction de RT-PCR a été faite avec un couple d'amplimers spécifiques et en prenant comme matrice l'ARN génomique viral extrait. L'ADN complémentaire amplifié a été extrait au phénol/chloroforme/alcool isoamylique (25:24:1) avant d'être digéré par les enzymes de restriction.

### Exemple 7 : plasmide pVR1012

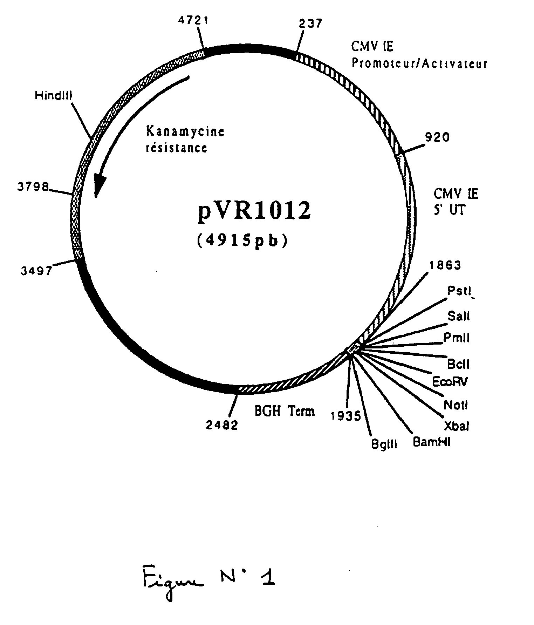

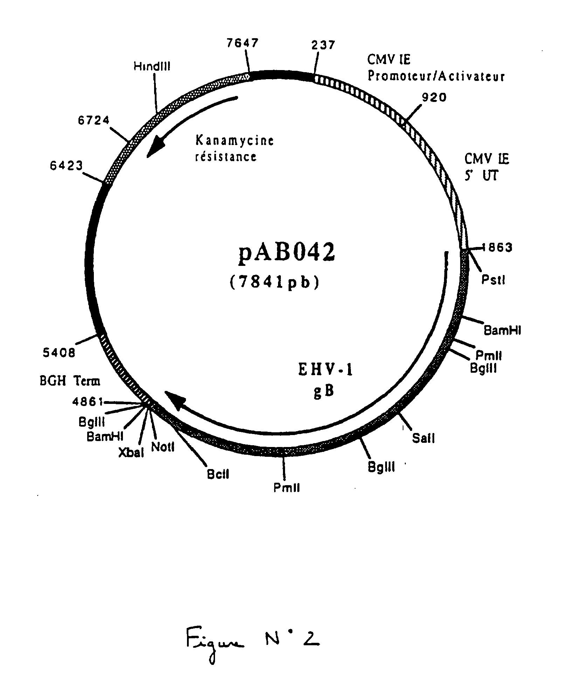

Le plasmide pVR1012 (Figure N° 1) a été obtenu auprès de Vical Inc. San Diego, CA, USA. Sa construction a été décrite dans J. Hartikka *et al*. (Human Gene Therapy. 1996. 7. 1205-1217).

### Exemple 8 : Construction du plasmide pAB042 (gène EHV-1 gB)

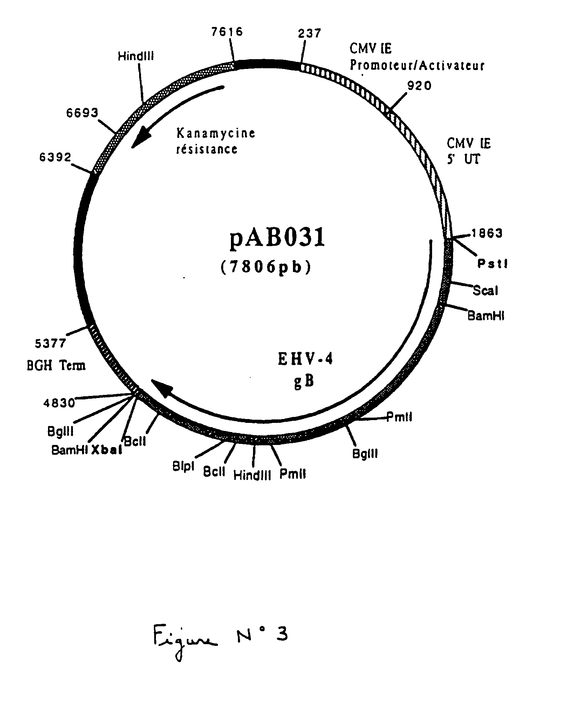

Une réaction de PCR a été réalisée avec l'ADN génomique de l'herpèsvirus équin de type 1 (EHV-1) (Souche Kentucky D) (P. Guo *et al.* J. Virol. 1990. **64.** 2399-2406) et avec les oligonucléotides suivants: 
AB013 (32 mer) (SEQ ID N° 1)
5'AAAACTGCAGCCGTCATGTCCTCTGGTTGCCG 3'
 
AB014 (39 mer) (SEQ ID N° 2)
5'ATAAGAAGCGGCCGCTAAACATGTTTAAACCATTTTTTC 3'
 pour isoler le gène codant pour la glycoprotéine gB (EHV-1 gB) sous la forme d'un fragment PstI-NotI. Après purification, le produit de PCR de 2981 pb a été digéré par *Pst*I et *Not*I pour isoler un fragment PstI-NotI de 2959 pb. Ce fragment a été ligaturé avec le vecteur pVR1012 (exemple 7), préalablement digéré avec *Pst*I et *Not*I, pour donner le plasmide pAB042 (7841 pb) (Figure N° 2).

### Exemple 9 : Construction du plasmide pAB031 (gène EHV-4 gB)

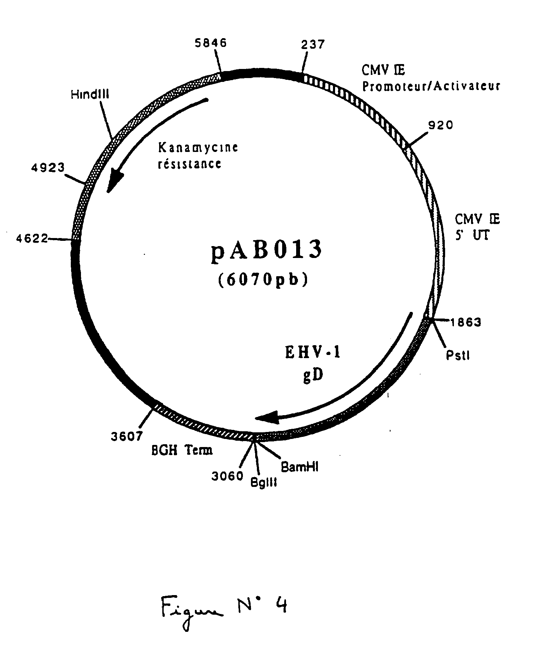

Une réaction de PCR a été réalisée avec l'ADN génomique de l'herpèsvirus équin de type 4 (EHV-4) (Souche 1942) (M. Riggio *et al.* J. Virol. 1989. 63. 1123-1133) et avec les oligonucléotides suivants: 
AB071 (38 mer) (SEQ ID N° 3)
5'AAAACTGCAGACATGTCCACTTGTTGCCGTGCTATTTG 3'
 AB074 (36 mer) (SEQ ID Nº 4) 5'CTAGTCTAGATTAAACCATTTTTCGCTTTCCATGG 3' pour amplifier un fragment de 2949 pb contenant le géne codant pour la glycoprotéine gB du EHV-4 sous la forme d'un fragment PstI-XbaI. Après purification, le produit de PCR a été digéré par *Pst*I et *Xbal* pour donner un fragment PstI-XbaI de 2931 pb.
Ce fragment a été ligaturé avec le vecteur pVR1012 (exemple 7), préalablement digéré avec Pstl et *Xbal,* pour donner le plasmide pAB031 (7806 pb) (Figure N° 3).

### Exemple 10 : Construction du plasmide pAB013 (gène EHV-1 gD)

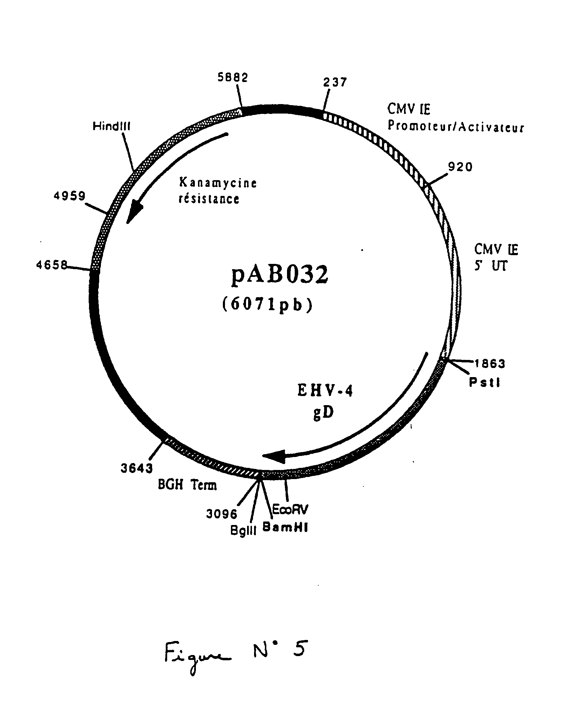

Une réaction de PCR a été réalisée avec l'ADN génomique de l'herpèsvirus équin de type 1 (EHV-1) (Souche Kentucky D) (J.C. Audonnet *et al*. J. Gen. Virol. 1990. 71. 2969-2978) et avec les oligonucléotides suivants: 
AB030 (32 mer) (SEQ ID N° 5)
5'AAAACTGCAGCATGTCTACCTTCAAGCTTATG 3'
 
AB031 (37 mer) (SEQ ID N° 6)
5'CGCGGATCCTTACGGAAGCTGGGTATATTTAACATCC 3'
 pour isoler le gène codant pour la glycoprotéine gD (EHV-1 gD) sous la forme d'un fragment PstI-BamHI. Après purification, le produit de PCR de 1228 pb a été digéré par *Pstl* et *Bam*HI pour isoler un fragment PstI-BamHI de 1211 pb. Ce fragment a été ligaturé avec le vecteur pVR1012 (exemple 7), préalablement digéré avec *Pst*I et *Bam*HI, pour donner le plasmide pAB013 (6070 pb) (Figure N° 4).

### Exemple 11 : Construction du plasmide pAB032 (gène EHV-4 gD)

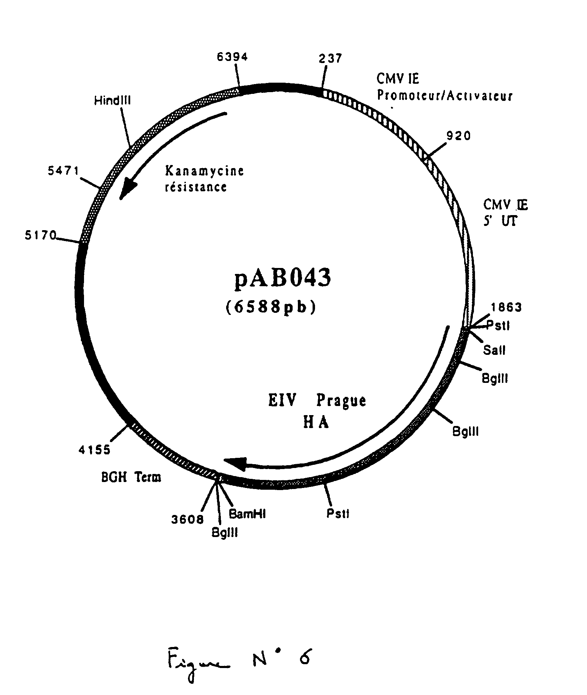

Une réaction de PCR a été réalisée avec l'ADN génomique de l'herpèsvirus équin de type 4 (EHV-4) (A. Cullinane *et al*. J. Gen. Virol. 1993. **74.** 1959-1964) et avec les oligonucléotides suivants: 
AB075 (33 mer) (SEQ ID N° 7)
5'AAAACTGCAGATATGTCTACCTTCAAGCCTATG 3'
 
AB076 (33 mer) (SEQ ID Nº 8)
5'CGCGGATCCTTACGGAAGCTGAGTATATTTGAC 3'
 pour isoler le gène codant pour la glycoprotéine gD du EHV-4 (EHV-4 gD) sous la forme d'un fragment PstI-BamHI. Après purification le produit de PCR de 1230 pb a été digéré par *Pst*I et *Bam*HI pour isoler un fragment PstI-BamHI de 1212 pb. Ce fragment a été ligaturé avec le vecteur pVR1012 (exemple 7), préalablement digéré avec *Pst*I et *Bam*HI, pour donner le plasmide pAB032 (6071 pb) (Figure N° 5).

### Exemple 12 : Construction du plasmide pAB043 (gène grippe équine HA souche Prague)

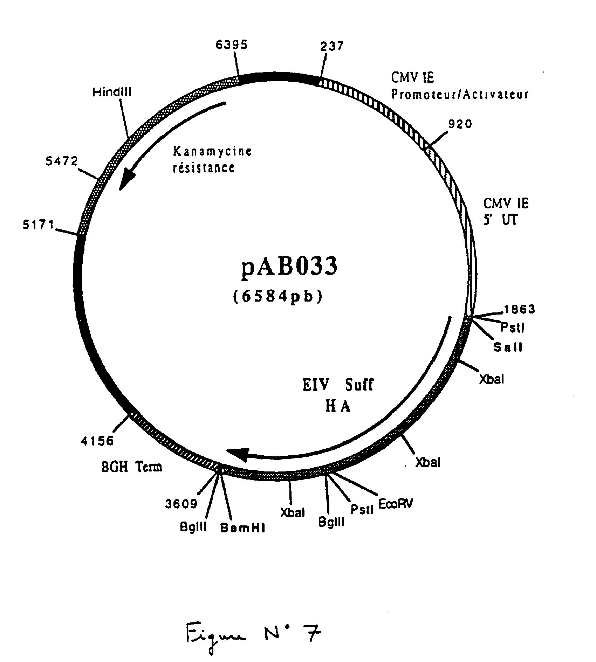

Une réaction de RT-PCR selon la technique décrite dans l'exemple 6 a été réalisée avec l'ARN génomique du virus de la grippe équine (Equine Influenza Virus ou EIV) (Souche H7N7 Prague) (J. Mc Cauley. N° d'accès séquence sur Genbank = X62552), préparé selon la technique décrite dans l'exemple 4, et. avec les oligonucléotides suivants: 
AB015 (36 mer) (SEQ ID N° 9)
5'ACGCGTCGACATGAACACTCAAATTCTAATATTAGC 3'
 
AB016 (35 mer) (SEQ ID N° 10)
5'CGCGGATCCCTTATATACAAATAGTGCACCGCATG 3'
 pour isoler le gène codant pour la glycoprotéine HA du virus de la grippe équine sous la forme d'un fragment SalI-BamHI. Après purification, le produit de RT-PCR de 1733 pb a été digéré par *Sal*I et *Bam*HI pour isoler un fragment Sall-BamHI de 1720 pb. Ce fragment a été ligaturé avec le vecteur pVR1012 (exemple 7), préalablement digéré avec *Sal*I et *Bam*HI, pour donner le plasmide pAB043 (6588 pb) (Figure N° 6).

### Exemple 13 : Construction du plasmide pAB033 (gène Grippe équine HA souche Suffolk)

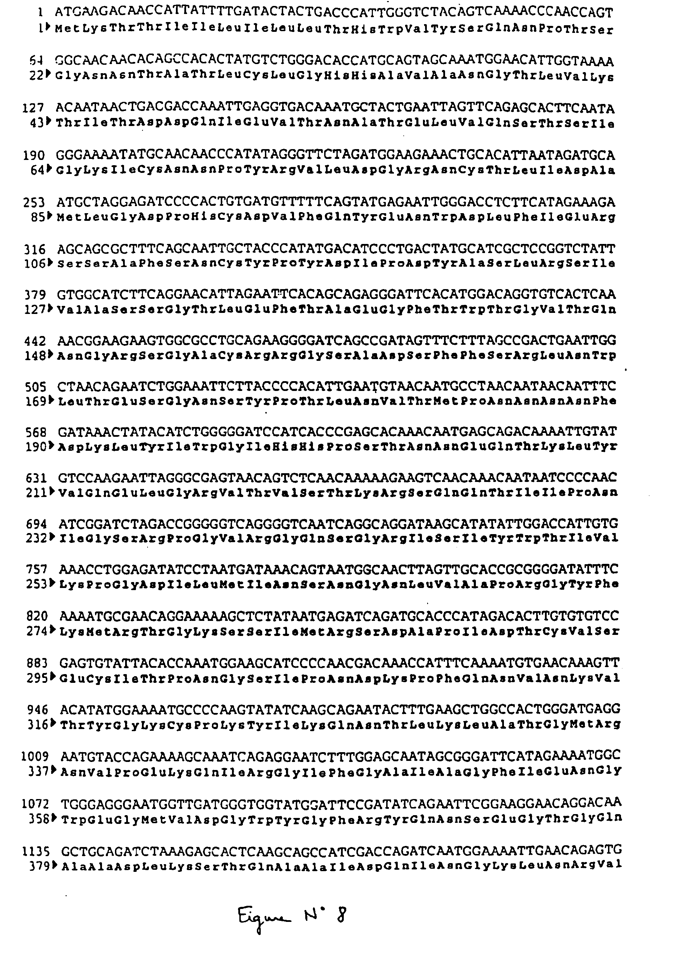

Une réaction de RT-PCR selon la technique de l'exemple 6 a été réalisée avec l'ARN génomique du virus de la grippe équine (EIV) (Souche Suffolk) (M. Binns. N° d'accès séquence sur Genbank = X68437), préparé selon la technique de l'exemple 4, et avec les oligonucléotides suivants: AB077 (33 mer) (SEQ ID N° 11) 5'ACGCGTCGACGCATGAAGACAACCATTATTTTG 3' 
AB078 (34 mer) (SEQ ID N° 12)
5'CGCGGATCCTCAAATGCAAATGTTGCATCTGATG 3'
 pour isoler le gène codant pour la glycoprotéine HA du virus de la grippe équine sous la forme d'un fragment Sall-BamHl. Après purification, le produit de RT-PCR de 1729 pb a été digéré par *Sal*I et *Bam*HI pour isoler un fragment Sall-BamHl de 1717 pb. Ce fragment a été ligaturé avec le vecteur pVR1012 (exemple 7), préalablement digéré avec *Sal*I et *Bam*HI, pour donner le plasmide pAB033 (6584 pb) (Figure N° 7).

### Exemple 14 : Construction du plasmide pAB099 (gène Grippe équine HA souche Fontainebleau)

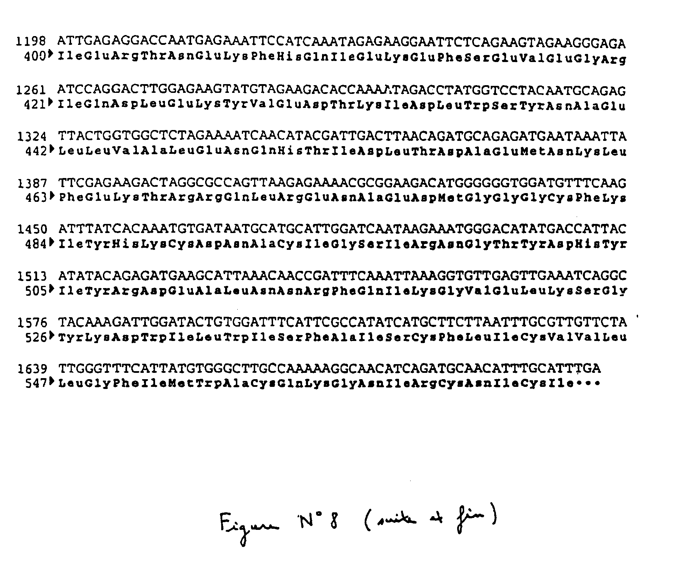

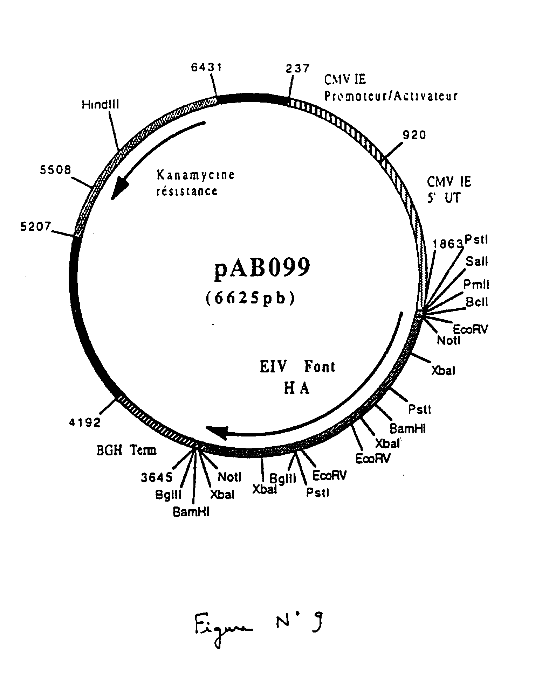

Une réaction de RT-PCR selon l'exemple 6 a été réalisée avec l'ARN génomique. du virus de la grippe équine (EIV) (Souche Fontainebleau), préparé selon l'exemple 4, et avec les oligonucléotides suivants: 
AB186 (32 mer) (SEQ ID N° 13)
5'TTTGCGGCCGCATGAAGACAACCATTATTTTG 3'
 
AB187 (35 mer) (SEQ ID N° 14)
5'TTTGCGGCCGCTTACTCAAATGCAAATGTTGCATC 3'
 pour isoler le gène codant pour la glycoprotéine HA du virus de la grippe équine (souche Fontainebleau) (Figure N° 8 et SEQ ID N° 15) sous la forme d'un fragment NotI-NotI. Après purification le produit de RT-PCR de 1724 pb a été digéré par *Not*I pour isoler un fragment NotI-NotI de 1710 pb. Ce fragment a été ligaturé avec le vecteur pVR1012 (exemple 7), préalablement digéré avec *Not*I, pour donner le plasmide pAB099 (6625 pb) qui contient le gène HA (grippe équine souche Fontainebleau) dans la bonne orientation par rapport au promoteur (Figure N° 9).

### Exemple 15 : Construction du plasmide pAB085 (gène Grippe équine NP souche Prague)

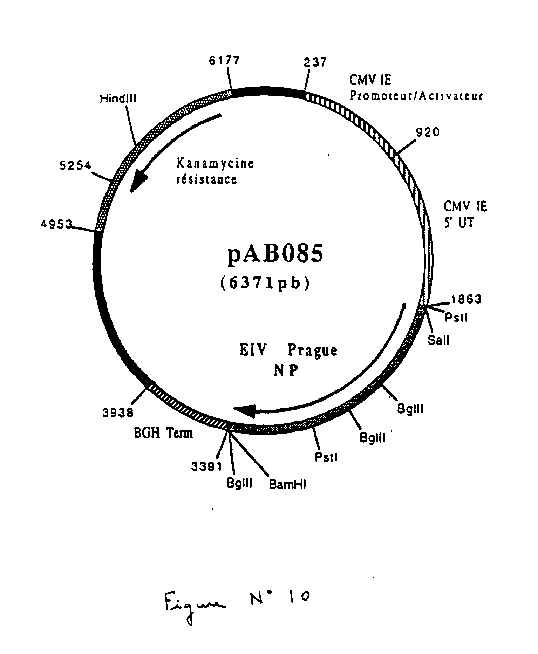

Une réaction de RT-PCR selon la technique de l'exemple 6 a été réalisée avec l'ARN génomique du virus de la grippe équine (EIV) (Souche H7N7 Prague) (O. Gorman *et al*. J. Virol. 1991. **65.** 3704-3714), préparé selon la technique de l'exemple 4, et avec les oligonucléotides suivants: 
AB156 (32 mer) (SEQ ID N° 16)
5'CCGGTCGACATGGCGTCTCAAGGCACCAAACG 3'
 
AB159 (34 mer) (SEQ ID N° 17)
5'CGCGGATCCTTAATTGTCAAACTCTTCTGCATTG 3'
 pour isoler le gène codant pour la nucléoprotéine NP du virus de la grippe équine sous la forme d'un fragment SalI-BamHI. Après purification, le produit de RT-PCR de 1515 pb a été digéré par *Sal*I et *Bam*HI pour isoler un fragment SalI-BamHI de 1503 pb. Ce fragment a été ligaturé avec le vecteur pVR1012 (exemple 7), préalablement digéré avec *Sal*I et *Bam*HI, pour donner le plasmide pAB085 (6371 pb) (Figure N° 10).

### Exemple 16 : Construction du plasmide pAB084 (gène Grippe équine NP souche Jillin)

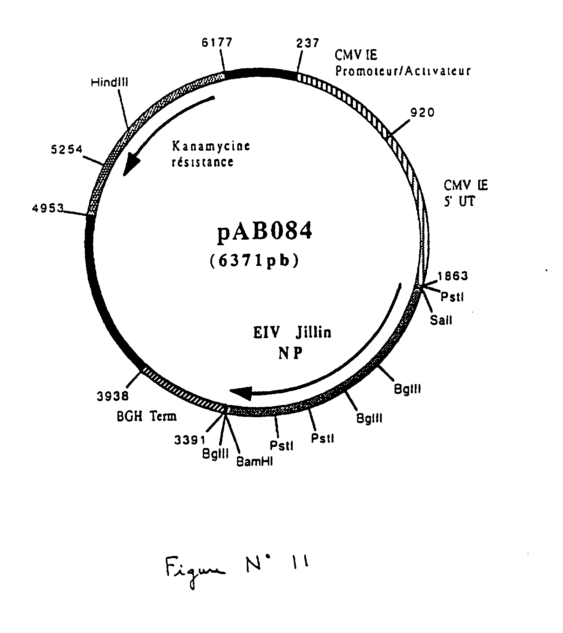

Une réaction de RT-PCR selon la technique de l'exemple 6 a été réalisée avec l'ARN génomique du virus de la grippe équine (EIV) (Souche H3N8 Jillin) (O. Gorman *et al.* J. Virol. 1991. 65. 3704-3714), préparé selon la technique de l'exemple 4, et avec les oligonucléotides suivants: 
AB156 (32 mer) (SEQ ID N° 16)
5'CCGGTCGACATGGCGTCTCAAGGCACCAAACG 3'
 
AB157 (34 mer) (SEQ ID N° 18)
5'CGCGGATCCTTAATTGTCATATTCCTCTGCATTG 3'
 pour isoler le gène codant pour la nucléoprotéine NP du virus de la grippe équine sous la forme d'un fragment SalI-BamHI. Après purification, le produit de RT-PCR de 1515 pb a été digéré par *Sal*I et *Bam*HI pour isoler un fragment Sall-BamHl de 1503 pb. Ce fragment a été ligaturé avec le vecteur pVR1012 (exemple 7), préalablement digéré avec Sail et BamHI, pour donner le plasmide pAB084 (6371 pb) (Figure N° 11).

### Exemple 17 : Construction du plasmide pAB070 (gène sous-unité C toxine tétanique)

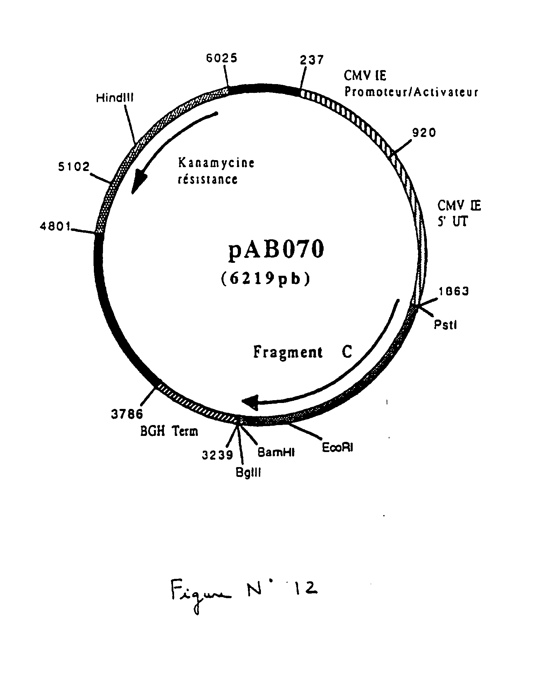

Une réaction de PCR a été réalisée avec l'ADN génomique de *Clostridium tetani* (Souche CN3911) (N. Fairweather *et al*. J. Bact. 1986. 165. 21-27), préparé selon la technique de l'exemple 2, et avec les oligonucléotides suivants: 
AB128 (34 mer) (SEQ ID N° 19)
5'AAACTGCAGATGAAAAATCTGGATTGTTGGGTTG 3'
 
AB129 (30 mer) (SEQ ID N° 20)
5'TTTGGATCCTTAATCATTTGTCCATCCTTC 3'
 pour isoler la séquence codant pour la sous-unité C de la toxine de *Clostridium tetani sous* la forme d'un fragment PstI-BamHI. Après purification, le produit de PCR de 1377 pb a été digéré par *Pstl* et *Bam*HI pour isoler un fragment Pstl-BamHl de 1361 pb. Ce fragment a été ligaturé avec le vecteur pVR1012. (exemple 7), préalablement digéré avec *Pst*I et *Bam*HI, pour donner le plasmide pAB070 (6219 pb) (Figure N° 12).

### Exemple 18 : Construction du plasmide pAB017 (gène ospA de Borrelia burgdorferi)

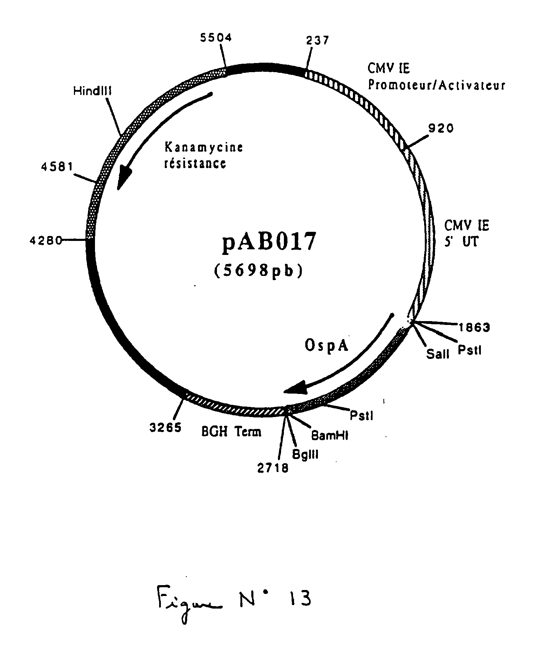

Une réaction de PCR a été réalisée avec l'ADN génomique de *Borrelia burgdorferi* (Souche B31) (S. Bergstrom *et al*. Mol. Microbiol. 1989. 3. 479-486), préparé selon la technique de l'exemple 2, et avec les oligonucléotides suivants: 
AB038 (37 mer) (SEQ ID N° 21)
5'ACGCGTCGACTATGAAAAAATATTTATTGGGAATAGG 3'
 
AB039 (34 mer) (SEQ ID N° 22)
5'CGCGGATCCCTTATTTTAAAGCGTTTTTAATTTC 3'
 pour isoler le gène codant pour la protéine de membrane OspA sous la forme d'un fragment Sall-BamHl. Après purification, le produit de PCR de 842 pb a été digéré par *Sal*I et *Bam*HI pour isoler un fragment Sall-BamHl de 829 pb. Ce fragment a été ligaturé avec le vecteur pVR1012 (exemple 7), préalablement digéré avec Sall et BamHl, pour donner le plasmide pAB017 (5698 pb) (Figure N° 13).

### Exemple 19 : Construction du plasmide pAB094 (gène E2 du virus de l'encéphalite de l'Est)

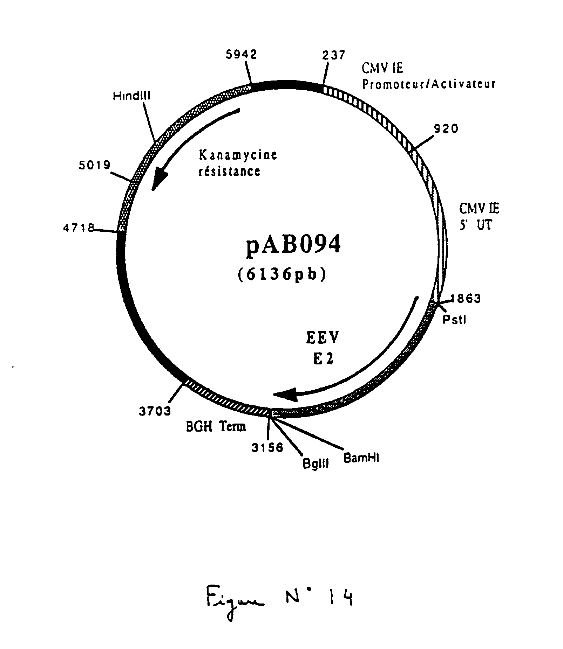

Une réaction de RT-PCR selon la technque de l'exemple 6 a été réalisée avec l'ARN génomique du virus de l'encéphalite de l'Est (EEV) (Souche North America 82V2137) (S. Weaver *et al*. Virology. 1993. **197.** 375-390), préparé selon la technique de l'exemple 4, et avec les oligonucléotides suivants: 
AB176 (34 mer) (SEQ ID N° 23)
5'AAACTGCAGATGGATTTGGACACTCATTTCACCC 3'
 
AB177 (44 mer) (SEQ ID N° 24)
5'CGCGGATCCTCAATAAAAATCATGCCCTCGTCGGCTTAATGCAG 3'
 pour isoler le gène codant pour la glycoprotéine E2 du EEV sous la forme d'un fragment PstI-BamHI. Après purification, le produit de RT-PCR de 1294 pb a été digéré par *Pst*I et *Bam*HI pour isoler un fragment PstI-BamHI de 1278 pb. Ce. fragment a été ligaturé avec le vecteur pVR1012 (exemple 7), préalablement digéré avec PstI et BamHI, pour donner le plasmide pAB094 (6136 pb) (Figure N° 14).

### Exemple 20 : Construction du plasmide pAB093 (gène C du virus de l'encéphalite de l'Est)

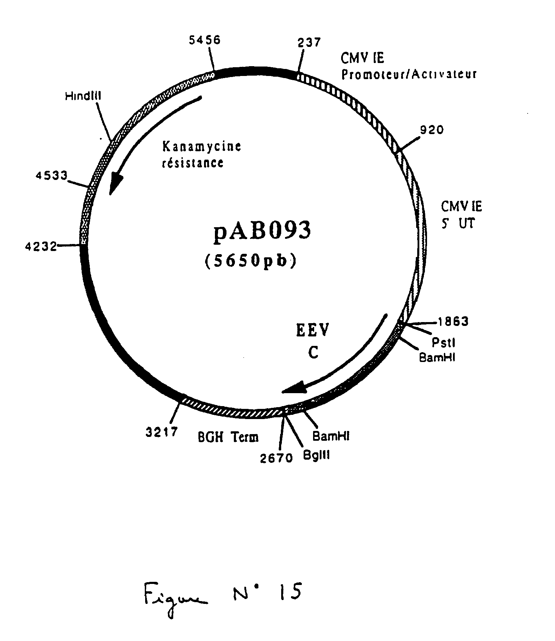

Une réaction de RT-PCR selon la technique de l'exemple 6 a été réalisée avec l'ARN génomique du virus de l'encéphalite de l'Est (EEV) (Souche North America 82V2137) (S. Weaver *et al*. Virology. 1993. 197. 375-390), préparé selon la technique de l'exemple 4, et avec les oligonucléotides suivants: 
AB174 (33 mer) (SEQ ID N° 25)
5'AAACTGCAGATGTTCCCATACCCTACACTTAAC 3'
 
AB175 (45 mer) (SEQ ID N° 26)
5'TGAAGATCTTCAATAAAAATCACCATGGCTCTGACCCCTCTGGTG 3'
 pour isoler le gène codant pour la protéine de capside C (EEV C) sous la forme d'un fragment PstI-BglII. Après purification, le produit de RT-PCR de 801 pb a été digéré par *Pst*I et *Bgl*II pour isoler un fragment PstI-BglII de 785 pb. Ce fragment a été ligaturé avec le vecteur pVR1012 (exemple 7), préalablement digéré avec *Pst*I et *Bgl*II, pour donner le plasmide pAB093·(5650 pb) (Figure N° 15).

### Exemple 21 : Construction du plasmide pAB096 (gène E2 du virus de l'encéphalite de l'Ouest)

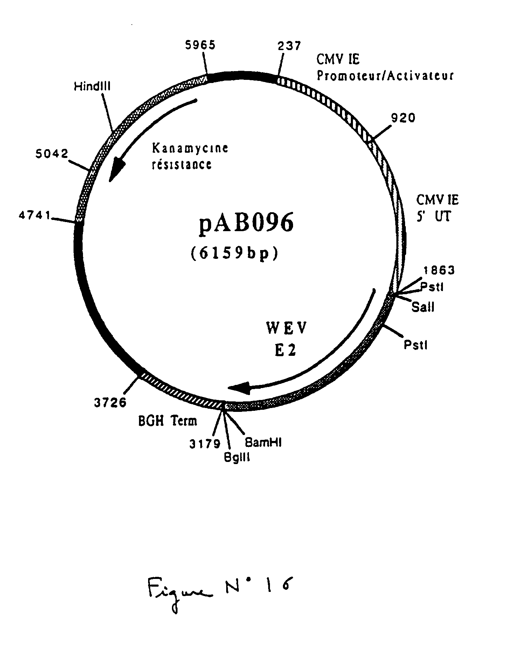

Une réaction de RT-PCR selon la technique de l'exemple 6 a été réalisée avec l'ARN génomique du virus de l'encéphalite de l'Ouest (WEV) (Souche BSF1703) (C. Hahn *et al*. Proc. Natl. Acad. Sci. USA. 1988. **85.** 5997-6001), préparé selon la technique de l'exemple 4, et avec les oligonucléotides suivants: 
AB180 (35 mer) (SEQ ID N° 27)
5'ACGCGTCGACATGAGCATTACCGATGACTTCACAC 3'
 
AB181 (44 mer) (SEQ ID N° 28)
5'CGCGGATCCTCAATAAAAATCAAGCGTTGGTTGGCCGAATACAG 3'
 pour isoler le gène codant pour la glycoprotéine E2 du WEV sous la forme d'un fragment SalI-BamHI. Après purification, le produit de RT-PCR de 1304 pb a été digéré par *Sal*I et *Bam*HI pour isoler un fragment Sall-BamHl de 1291 pb. Ce fragment a été ligaturé avec le vecteur pVR1012 (exemple 7), préalablement digéré avec *Sal*I et *Bam*HI, pour donner le plasmide pAB096 (6159 pb) (Figure N° 16).

### Exemple 22 : Construction du plasmide pAB095 (gène C du virus de l'encéphalite de l'Ouest)

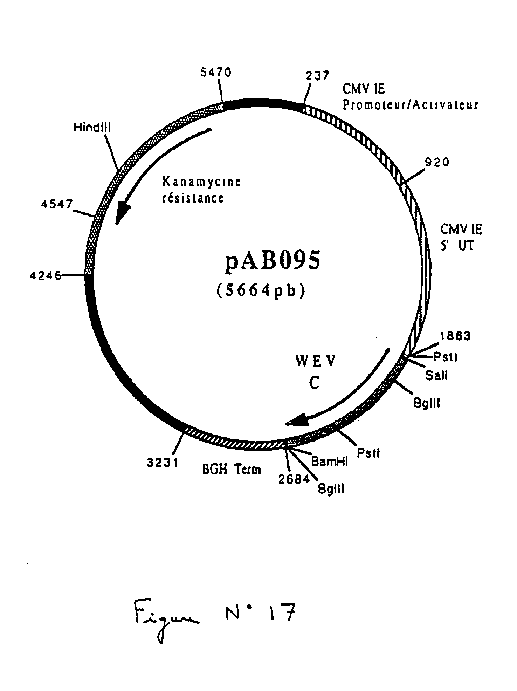

Une réaction de RT-PCR selon l'exemple 6 a été réalisée avec l'ARN génomique du virus de l'encéphalite de l'Ouest (WEV) (Souche BSF1703) (C. Hahn *et al*. Proc.Natl.Acad.Sci.USA. 1988. **85.** 5997-6001), préparé selon l'exemple 4, et avec tes oligonucléotides suivants: 
AB178 (34 mer) (SEQ ID N° 29)
5'ACGCGTCGACATGTTTCCATACCCTCAGCTGAAC 3'
 
AB179 (44 mer) (SEQ ID N° 30)
5'CGCGGATCCTCAATAAAAATCACCACGGTTCAGAACCTTCGGGG 3'
 pour isoler le gène codant pour la protéine de capside C du virus WEV sous la forme d'un fragment Sall-BamHl. Après purification, le produit de RT-PCR de 809 pb a été digéré par *Sal*I et *Bam*HI pour isoler un fragment SalI-BamHI de 796 pb. Ce fragment a été ligaturé avec le vecteur pVR1012 (exemple 7), préalablement digéré avec *Sal*I et *Bam*HI, pour donner le plasmide pAB095 (5664 pb) (Figure N° 17).

### Exemple 23 : Construction du plasmide pAB098 (gène E2 du virus de l'encéphalite vénézuélienne)

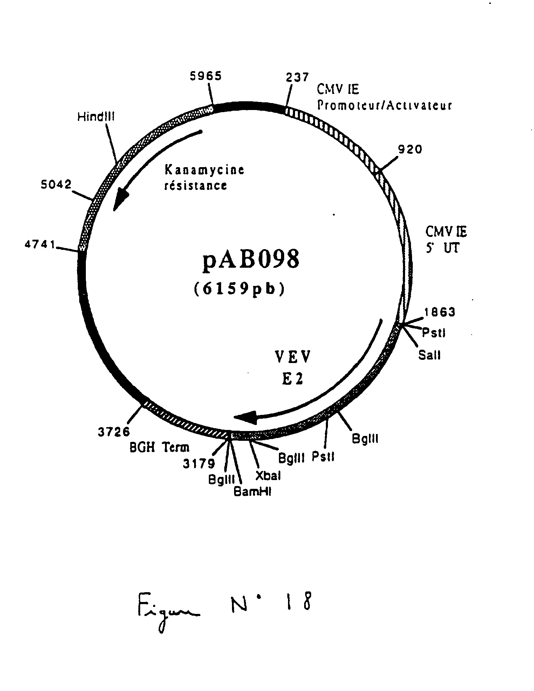

Une réaction de RT-PCR selon l'exemple 6 a été réalisée avec l'ARN génomique du virus de l'encéphalite vénézuélienne (VEV) (Souche P676 (Type IC)) (R. Kinney *et al*. Virology. 1992. 191. 569-580), préparé selon l'exemple 4, et avec les oligonucléotides suivants: 
AB184 (35 mer) (SEQ ID N° 31)
5'ACGCGTCGACATGTCCACCGAGGAGCTGTTTAAGG 3'
 
AB185 (44 mer) (SEQ ID N° 32)
5'CGCGGATCCTCAATAAAAATCAGGCCCGGGCAGTGCGGGCGCAG 3'
 pour isoler le gène codant pour la glycoprotéine E2 du virus VEV sous la forme d'un fragment SalI-BamHI. Après purification, le produit de RT-PCR de 1304 pb a été digéré par *Sal*I et *Bam*HI pour isoler un fragment SalI-BamHI de 1291 pb. Ce fragment a été ligaturé avec le vecteur pVR1012 (exemple 7), préalablement digéré avec *Sal*I et *Bam*HI, pour donner le plasmide pAB098 (6159 pb) (Figure N° 18).

### Exemple 24 : Construction du plasmide pAB097 (gène C du virus de l'encéphalite vénézuélienne)

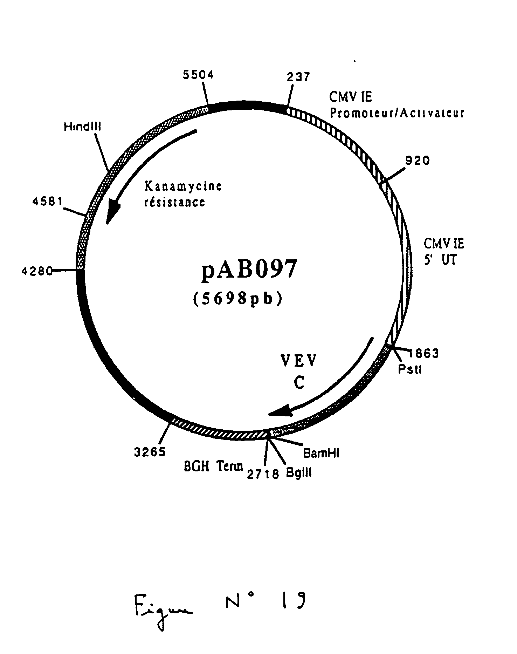

Une réaction de RT-PCR selon l'exemple 6 a été réalisée avec l'ARN génomique du virus de l'encéphalite vénézuélienne (VEV) (Souche P676 (Type IC)) (R. Kinney *et al*. Virology. 1992. 191. 569-580), prépaé selon l'exemple 4, et avec les oligonucléotides suivants: 
AB182 (30 mer) (SEQ ID N° 33)
5'AAACTGCAGATGTTCCCGTTCCAGCCAATG 3'
 
AB183 (45 mer) (SEQ ID N° 34)
5'CGCGGATCCTCAATAAAAATCACCATTGCTCGCAGTTCTCCGGAG 3'
 pour isoler le gène codant pour la protéine de capside C du virus VEV sous la forme d'un fragment PstI-BamHI. Après purification, le produit de RT-PCR de 856 pb a été digéré par *Pstl* et *Bam*HI pour isoler un fragment PstI-BamHI de 839 pb. Ce fragment a été ligaturé avec le vecteur pVR1012 (exemple 7), préalablement digéré avec *Pst*I et *Bam*HI, pour donner le plasmide pAB097 (5698 pb) (Figure N° 19).

### Exemple 25 : Construction du plasmide pAB041 (gène G du virus de la rage)

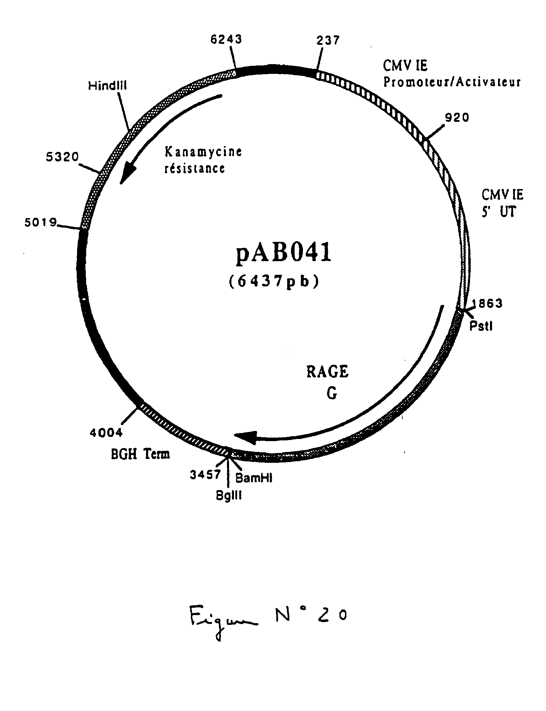

Une réaction RT-PCR selon l'exemple 6 a été réalisée avec l'ARN génomique du virus de la rage (Souche ERA) (A. Anilionis *et al*. Nature. 1981. 294. 275-278), préparé selon l'exemple 4, et avec les oligonucléotides suivants: 
AB011 (33 mer) (SEQ ID N° 35)
5'AAAACTGCAGAGATGGTTCCTCAGGCTCTCCTG 3'
 
AB012 (34 mer) (SEQ ID N° 36)
5'CGCGGATCCTCACAGTCTGGTCTCACCCCCACTC 3'
 pour amplifier un fragment de 1589 pb contenant le gène codant pour la protéine G du virus de la rage. Après purification, le produit de RT-PCR a été digéré par *Pstl* et *Bam*HI pour donner un fragment PstI-BamHI de 1578 pb. Ce fragment a été ligaturé avec le vecteur pVR1012 (exemple 7), préalablement digéré avec *Pst*I et *Bam*HI, pour donner le plasmide pAB041 (6437 pb) (Figure N° 20).

### Exemple 26 : Préparation et purification des plasmides

Pour la préparation des plasmides destinés à la vaccination des animaux, on peut utiliser toute technique permettant d'obtenir une suspension de plasmides purifiés majoritairement sous forme superenroulée. Ces techniques sont bien connues de l'homme de l'art. On peut citer en particulier la technique de lyse alcaline suivie de deux ultracentrifugations successives sur gradient de chlorure de césium en présence de bromure d'éthidium telle que décrite dans J. Sambrook *et al*. (*Molecular Cloning: A Laboratory Manual*. 2^{nd} Edition. Cold Spring Harbor Laboratory. Cold Spring Harbor. New York. 1989). On peut se référer également aux demandes de brevet PCT WO 95/21250 et PCT WO 96/02658 qui décrivent des méthodes pour produire l'échelle industrielle des plasmides utilisables pour la vaccination. Pour les besoins de la fabrication des vaccins (voir exemple 17), les plasmides purifiés sont resuspendus de manière à obtenir des solutions à haute concentration (> 2 mg/ml) compatibles avec le stockage. Pour ce faire, les plasmides sont resuspendus soit en eau ultrapure, soit en tampon TE (Tris-HCl 10 mM; EDTA 1 mM, pH 8,0).

### Exemple 27 : Fabrication des vaccins associés

Les divers plasmides nécessaires à la fabrication d'un vaccin associé sont mélangés à partir de leurs solutions concentrées (exemple 16). Les mélanges sont réalisés de telle manière que la concentration finale de chaque plasmide corresponde à la dose efficace de chaque plasmide. Les solutions utilisables pour ajuster la concentration finale du vaccin peuvent être soit une solution NACI à 0,9 % , soit du tampon PBS.
Des formulations particulières telles que les liposomes, les lipides cationiques, peuvent aussi être mises en oeuvre pour la fabrication des vaccins.

### Exemple 28 : Vaccination des chevaux

Les chevaux sont vaccinés avec des doses de 100 µg, 250 µg ou 500 µg par plasmide.
Les injections peuvent être réalisées à l'aiguille par voie intramusculaire dans les muscles du cou. Dans ce cas, les doses vaccinales sont administrées sous un volume de 2 ml.
Les injections peuvent être réalisées par voie intradermique en utilisant un appareil d'injection à jet liquide (sans aiguille) délivrant une dose de 0,2 ml en 5 points (0,04 ml par point d'injection) (par exemple, appareil "PIGJET"). Dans ce cas, les doses vaccinales sont administrées sous des volumes de 0,2 ou 0,4 ml, ce qui correspond respectivement à une ou à deux administrations. Lorsque deux administrations successives sont pratiquées au moyen de l'appareil PIGJET, ces administrations sont réalisées de manière décalée, de façon à ce que les deux zones d'injection soient séparées l'une de l'autre par une distance d'environ 1 à 2 centimètres.

## Revendications

1. Vaccin pour des équidés **caractérisé en ce qu'**il comprend un plasmide intégrant, de manière à l'exprimer in vivo dans les cellules, les gènes codant pour les antigènes C et E2 du virus de l'encéphalite.

2. Le vaccin selon la revendication 1, **caractérisé en ce que** le plasmide intègre de manière à l'exprimer in vivo dans les cellules le gène codant pour l'antigène E2 du virus de l'encéphalite.

3. Le vaccin selon l'une quelconque des revendications 1 à 2, **caractérisé en ce que** le virus de l'encéphalite est sélectionné dans le groupe consistant en virus de l'encéphalite de l'est EEV, virus de l'encéphalite de l'ouest WEV et virus de l'encéphalite vénézuélienne VEV.

4. Le vaccin selon la revendication 3, **caractérisé en ce qu'**il comprend un plasmide intégrant de manière à l'exprimer in vivo dans les cellules un gène codant pour l'antigène E2 d'un ou plusieurs virus de l'encéphalite sélectionné dans le groupe consistant en virus de l'encéphalite de l'est, virus de l'encéphalite de l'ouest et virus de l'encéphalite vénézuélienne.

5. Le vaccin selon l'une quelconque des revendications 1 à 4, **caractérisé en ce que** l'association des gènes codant pour plusieurs antigènes est réalisée par le mélange de plasmides exprimant un seul antigène.

6. Le vaccin selon l'une quelconque des revendications 1 à 4, **caractérisé en ce que** l'association des gènes codant pour plusieurs antigènes est réalisée en insérant plusieurs gènes dans un même plasmide.

7. Le vaccin selon la revendication 6, **caractérisé en ce que** les gènes sont présentés dans la même unité de transcription ou dans deux unités différentes.

8. Le vaccin selon l'une quelconque des revendications 1 à 7, **caractérisé en ce que** le plasmide comprend le promoteur précoce du cytomégalovirus CMV-IE.

9. Le vaccin selon l'une quelconque des revendications 1 à 7, **caractérisé en ce que** le plasmide comprend un promoteur sélectionné dans le groupe consistant en promoteur précoce du virus SV40, promoteur tardif du virus SV40, promoteur LTR du virus du Sarcome de Rous, promoteur de la desmine et promoteur de l'actine.

10. Le vaccin selon l'une quelconque des revendications 1 à 9, **caractérisé en ce qu'**il comprend de 10 ng à 1 mg par type de plasmide.

11. Le vaccin selon la revendication 10, **caractérisé en ce qu'**il comprend de 100 ng à 500 µg par type de plasmide.

12. Le vaccin selon la revendication 11, **caractérisé en ce qu'**il comprend de 1 µg à 250 µg par type de plasmide.

13. Utilisation d'un ou plusieurs plasmide(s) tels que décrit dans l'une quelconque des revendications 1 à 12, pour la fabrication d'un vaccin destiné à vacciner les équidés primo-vaccinés au moyen d'un premier vaccin sélectionné dans le groupe consistant en vaccin entier vivant, vaccin entier inactivé, vaccin de sous-unité, vaccin recombinant, ce premier vaccin présente le ou les antigène(s) assurant une protection croisée.

14. Kit de vaccination des équidés regroupant un vaccin selon l'une quelconque des revendications 1 à 12 et un vaccin de primo-vaccination sélectionné dans le groupe consistant en vaccin entier vivant, vaccin entier inactivé, vaccin de sous-unité, vaccin recombinant, ce vaccin de primo-vaccination présente le ou les antigène(s) assurant une protection croisée.
